# EUROPEAN PATENT SPECIFICATION

(11) **EP 4 213 910 B1**
(45) Date of publication and mention of the grant of the patent: **28.10.2026**
(21) Application number: 21789969.9
(22) Date of filing: 16.09.2021
(51) Int. Cl.: A61M 1/36, A61B 17/11

(54) **RETRIEVABLE ENDOVASCULAR IMPLANT FOR FISTULA CREATION AND MATURATION**
RÜCKHOLBARES ENDOVASKULÄRES IMPLANTAT ZUR FISTELERZEUGUNG UND -REIFUNG
IMPLANT ENDOVASCULAIRE RÉCUPÉRABLE POUR LA CRÉATION ET LA MATURATION DE FISTULES

(30) Priority: 16.09.2020 US 202063079087 P
(43) Date of publication of application: 26.07.2023
(73) Proprietor: C. R. Bard, Inc., Franklin Lakes, NJ 07417 (US)
(72) Inventor: TESSMER, Alexander, W., Phoenix, AZ 85018 (US); CHANDUSZKO, Andrzej, J., Chandler, AZ 85224 (US)
(74) Representative: Hoffmann Eitle
(86) International application number: PCT/US2021/050592
(87) International publication number: WO 2022/060933

(56) References cited:
- US-A1- 2003 088 256
- US-A1- 2015 157 475
- US-A1- 2019 015 104
- US-B2- 10 456 239

## Description

### TECHNICAL FIELD

This disclosure pertains to medical apparatuses and, in particular, to a retrievable endovascular implant for fistula creation and maturation.

### BACKGROUND

Currently, the only option for creation of an arteriovenous (AV) fistula in the United States for end-stage renal disease (ESRD) patients requiring dialysis is to undergo invasive surgery. It is estimated that just over 70,000 AV fistulas were created in 2016.

Fistula maturation is a process by which a fistula becomes suitable for cannulation and the criteria is the following: blood flow rate greater than 600ml/min, diameter greater than 6mm, depth less than 0.6cm beneath skin and the aforementioned criteria should be met in 4-6 weeks after surgical creation. Fistula maturation continues to be a problem with approximately 50% of fistulas failing to mature. Possible reasons why fistulas fail to mature include inadequate dilation, accessory veins, stenosis, thrombosis, arterial/venous disease, and hypotension. Existing solutions include balloon angioplasty, obliteration of competing vessels, and biodegradable magnesium stents.

Accordingly, a device is needed that assists in maturing fistulas, expands the vessel providing adequate dilation, increases flow rate, and helps prevent stenosis. The device would allow for a physician to create an AV fistula, regardless of the distance between the vein and artery, via a percutaneous, minimally invasive technique that will greatly reduce vessel trauma from surgery, remove variability limiting the frequency of re-interventions, achieve better maturation success, and present more anatomical fistula options.

US 2019/0015104 A1 discloses a connector for fluid communication between two anatomical compartments through at least one anatomical wall, wherein the connector includes a neck adapted and configured to be positioned across the anatomical wall(s); and a secondary element for securing the neck across the anatomical wall(s). A method is also provided for coupling two anatomical walls using said connector.

### SUMMARY

According to the invention, there is provided an endovascular implant according to claim 1.

The dependent claims define preferred embodiments.

### BRIEF DESCRIPTION OF THE DRAWING FIGURES

Figure 1 is a top view of an implant according to one aspect of the disclosure;
Figure la is a side sectional view of an implant of Figure 1;
Figure 2 is a top plan view of a precursory for forming the implant including a retriever; and
Figures 4-6 are progressive schematic views illustrating a possible use of the implant to form a fistula.

### DETAILED DESCRIPTION

In the following detailed description, numerous specific details are set forth to provide a thorough understanding of the disclosed concepts. Those of ordinary skill in the art will know that the disclosed inventions may be practiced without these specific details. In other instances, well-known methods, procedures, components, or structures may not have been described in detail so as not to obscure the disclosed inventions.

The description provided below and in regard to the figures applies to all embodiments unless noted otherwise, and features common to each embodiment are similarly shown and numbered.

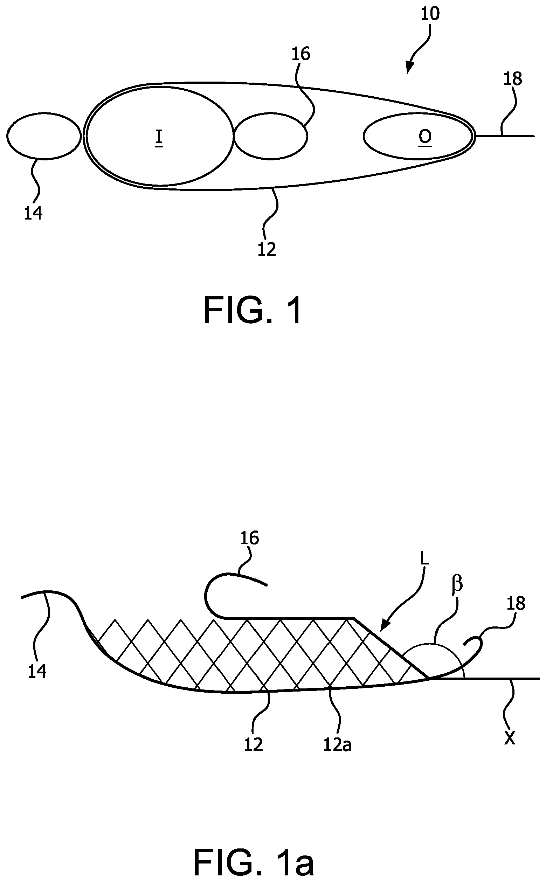

Referring to the drawing figures, an implant 10 is provided for forming an AV fistula, which is shown from both the top (Figure 1) and the side (Figure la). In the illustrated embodiment, the implant 10 comprises a single piece of tubing forming a tubular body 12. As illustrated in Figure la, the tubular body 12 may comprise a wall formed of a mesh comprising wires or struts defining a plurality of closed cells 12a, which thus provide the body with a significant degree of flexibility.

The body 12 may be at least partially covered by a coating or membrane formed of a biocompatible material (e.g., ePTFE, polyurethane, etc.). The covering may be self-sealing, such as after a needle puncture. As can be understood from Figures 1 and la, the so-coated body 12 thus forms a lumen L having a distal opening (inlet I) and a proximal opening (outlet O) for together passing a flow of fluid, such as blood, in an associated vessel once inserted therein, while also lending support for the corresponding vessel to maintain patency.

The implant 10 further includes one or more anchors 14, 16 for anchoring the implant in place in a desired location, such as to form an AV fistula. The anchors 14, 16 are shown on the distal end of the implant 10, which correspond to the arterial side at a fistula site. The anchors 14, 16 may be oval in shape, or may have different shapes, and may be close-ended or open-ended. The anchors 14, 16 may extend obliquely and in opposite directions from adjacent an open upper (inlet) end of the implant 10. While two anchors 14, 16 are shown, more than two may be provided. Anchors (not shown) may also optionally be provided on the proximal end of the implant 10, corresponding to the location in the vein in use.

The implant 10 also includes a retriever 18. In one embodiment, the retriever 18 takes the form of a hook, the details of which are outlined further in the following description. The hook forming the retriever 18 may be located adjacent a proximal (outlet O) end of the implant 10.

The hook forming the retriever 18 is connected to the body 12, such as directly to an elongated spine 20 extending longitudinally along one side (e.g., an underside) of the implant 10 in the illustrated orientation, and is bounded on both lateral sides by the closed cells 12a. This spine 20 is formed of material that is generally wider circumferentially than the wires or struts forming the closed cells 12a of the body 12, and thus enhances the ability of the implant 10 to be retrieved using the associated retriever 18. While only one spine 20 is shown, more than one may be present, if desired, or the spine may be omitted altogether.

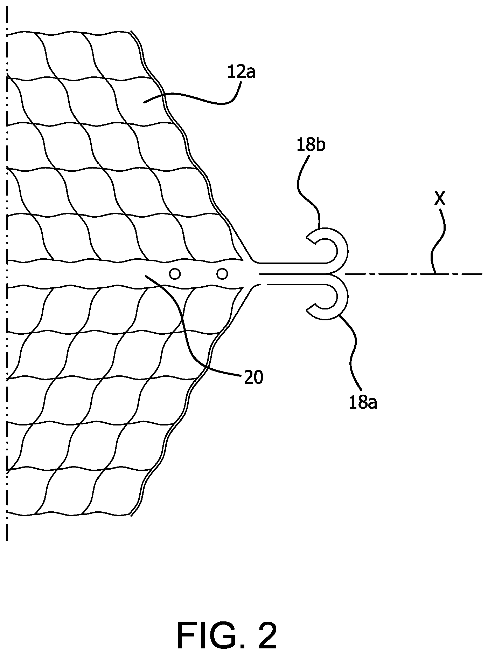

Formation of the implant 10 may be achieved by cutting the body 12 from a single piece of tubular material. Turning to the plan view of Figure 2, this processing allows for two longitudinally extending J-shaped hooks 18a, 18b to be cut directly from the single thickness material, which may be considered to create a preform for forming the implant 10 (and thus the hooks have a similar thickness to a wall of the body 12). A radially innermost portion of each hook 18a, 18b is offset from a central axis X of the body. The hooks 18a, 18b may thus be folded or bent/twisted into a juxtaposed, abutting relationship, and then bonded together (such as by a weld) to form a double thickness hook serving as the retriever 18 of Figures 1 and la. It can be appreciated that, in view of the forming process described above, the height of the hooks 18a, 18b may be substantially smaller in relation to the diameter of the body 12 of the implant 10, thus minimizing impedance to fluid flow.

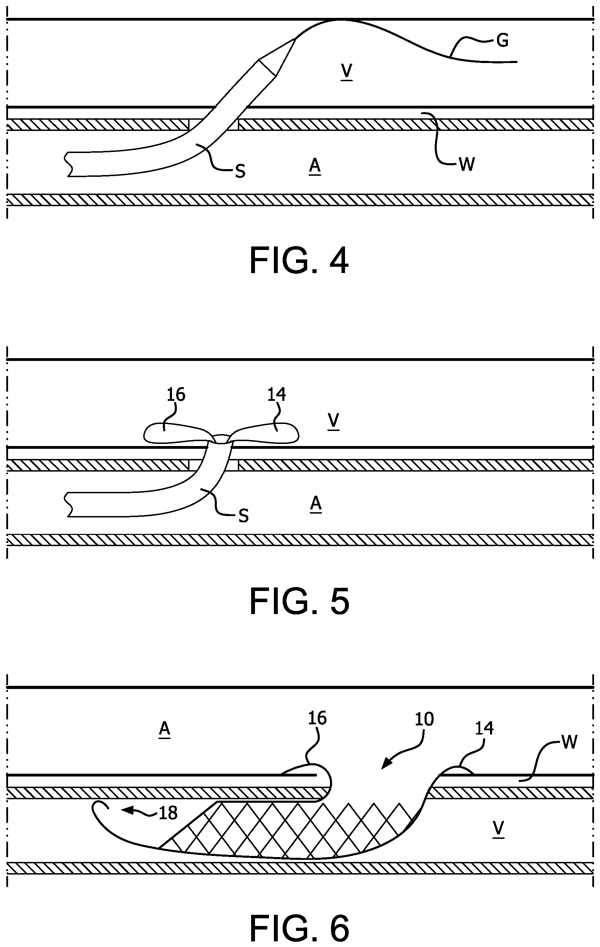

Turning now to Figures 3-5, use of the implant 10 to form an AV fistula is described. A sheath S may be used to deliver the implant, such as by delivering the sheath to a fistula creation site through a vein V and a needle (not shown) may be used to create (puncture) a hole through the venous and the arterial wall W. A guidewire G is then used to cross into the artery A through the needle.

The implant 10 may then be delivered to the tip of the sheath S. The sheath S is then retracted. Consequently, the distal anchors 14, 16 of the implant 10 are deployed, followed by the tubular body 12.

As a result, the lumen L provides a conduit for the arterial blood to flow into the vein, thus creating an AV fistula. Optionally, the vein V may be dilated with a PTA balloon prior to implantation to roughly match the implant diameter. The balloon may also be used to dilate the conduit created with the needle and the sheath prior to implantation or dilate the implant after its deployment.

After a particular time (e.g., several days or weeks), the implant 10 may be percutaneously removed. This may be done using a snare (not shown) to pull the implant longitudinally into a sheath. Turning back to Figure la, it can also be understood that at least the end associated with the retriever 18 may be sloped or tapered so as to form a second angle β with a horizontal axis X. As can be appreciated, when the retriever 18 is pulled longitudinally for purposes of retrieval, this taper allows for the narrower tapered portion of the implant 10 to initially enter the sheath when pulled toward the opening thereof. Further longitudinal movement into the sheath may thus cause a partial collapse of the implant 10 to ensure recovery, even if the inner diameter of the sheath is much smaller than the outer diameter of the implant 10.

The implant 10 may be made of a variety of materials. For example, the implant 10 may be fabricated from a shape memory material, such as Nitinol, or other metals/alloys, such as steel (e.g., stainless), nickel-chromium, cobalt-chromium alloys, titanium alloys, or the like. The implant 10 may also be formed of a polymer material, or even a bioabsorbable material, such as magnesium or other such materials, or composites of any materials.

The implant 10 may also be coated with a medicine or pharmacological agent (e.g., Paclitaxel) for treatment purposes, as is known in the art. The implant 10 may also be made radiopaque or incorporate radiopaque markers for identification using fluoroscopy.

Each of the following terms written in singular grammatical form: "a", "an", and "the", as used herein, means "at least one", or "one or more". Use of the phrase "one or more" herein does not alter this intended meaning of "a", "an", or "the". Accordingly, the terms "a", "an", and "the", as used herein, may also refer to, and encompass, a plurality of the stated entity or object, unless otherwise specifically defined or stated herein, or, unless the context clearly dictates otherwise. For example, the phrases: "a unit", "an apparatus", "an assembly", "a mechanism", "a component, "an element", and "a step or procedure", as used herein, may also refer to, and encompass, a plurality of units, a plurality of apparatuses, a plurality of assemblies, a plurality of mechanisms, a plurality of components, a plurality of elements, and, a plurality of steps or procedures, respectively.

Each of the following terms: "includes", "including", "has", "having", "comprises", and "comprising", and, their linguis- tic/grammatical variants, derivatives, or/and conjugates, as used herein, means "including, but not limited to", and is to be taken as specifying the stated component(s), feature(s), characteristic(s), parameter(s), integer(s), or step(s), and does not preclude addition of one or more additional com- ponent(s), feature(s), characteristic(s), parameter(s), integer(s), step(s), or groups thereof. Each of these terms is considered equivalent in meaning to the phrase "consisting essentially of." Each of the phrases "consisting of and "consists of, as used herein, means "including and limited to". The phrase "consisting essentially of' means that the stated entity or item (system, system unit, system sub-unit apparatus, assembly, sub-assembly, mechanism, structure, component element or, peripheral equipment utility, accessory, or material, method or process, step or procedure, sub-step or sub-procedure), which is an entirety or part of an exemplary embodiment of the disclosed invention, or/and which is used for implementing an exemplary embodiment of the disclosed invention, may include at least one additional feature or characteristic" being a system unit system subunit apparatus, assembly, sub-assembly, mechanism, structure, component or element or, peripheral equipment utility, accessory, or material, step or procedure, sub-step or sub-procedure), but only if each such additional feature or characteristic" does not materially alter the basic novel and inventive characteristics or special technical features, of the item.

The term "method", as used herein, refers to steps, procedures, manners, means, or/and techniques, for accomplishing a given task including, but not limited to, those steps, procedures, manners, means, or/and techniques, either known to, or readily developed from known steps, procedures, manners, means, or/and techniques, by practitioners in the relevant field(s) of the disclosed invention.

Terms of approximation, such as the terms about, substantially, approximately, etc., as used herein, refers to ± 10 % of the stated value. Use of the terms parallel or perpendicular are meant to mean approximately meeting this condition, unless otherwise specified.

It is to be fully understood that certain aspects, characteristics, and features, of the invention, which are, for clarity, illustratively described and presented in the context or format of a plurality of separate embodiments, may also be illustratively described and presented in any suitable combination or sub-combination in the context or format of a single embodiment. Conversely, various aspects, characteristics, and features, of the invention which are illustratively described and presented in combination or sub-combination in the context or format of a single embodiment may also be illustratively described and presented in the context or format of a plurality of separate embodiments.

Although the inventions of this disclosure have been illustratively described and presented by way of specific exemplary embodiments, and examples thereof, it is evident that many alternatives, modifications, or/and variations, thereof, will be apparent to those skilled in the art. For example, while a pair of hooks is described above, it can be appreciated that more than two hooks can be used to form the single hook, and thus retriever 18. Likewise, multiple single hooks, each formed of a double hook, can be provided on a single implant, if desired for a particular use.

## Claims

1. An endovascular implant (10) for forming a fistula, comprising:
a body (12) including a wall forming a lumen (L), the body including a distal end having a distal opening and a proximal end having a proximal opening;
an anchor (14, 16) coupled to the distal end of the body adjacent the distal opening; and
a retriever (18) coupled to the proximal end of the body adjacent the proximal opening;
wherein the wall comprises a material having a thickness, and **characterized by** having a pair of interconnected hooks (18a, 18b) forming the retriever (18), each hook of the pair of hooks having a first longitudinal portion extending from and unitarily formed with the wall and a second portion connected to the first longitudinal portion, each hook being formed of the material of the wall having the thickness.

2. The endovascular implant of claim 1, wherein the first longitudinal portion of each hook is partially twisted and the second portion projects radially inward from the first longitudinal portion.

3. The endovascular implant of claim 2, wherein the pair of hooks (18a, 18b) are at least partially connected together by a bond.

4. The endovascular implant of claim 3, wherein the bond comprises a weld.

5. The endovascular implant of claim 2, wherein the pair of interconnected hooks (18a, 18b) include a radially innermost portion offset from a central axis of the body (12).

6. The endovascular implant of claim 2, wherein the first longitudinal portion of each hook is partially twisted and the second portion projects radially inward from the first longitudinal portion.

7. The endovascular implant of any of claims 2-6, wherein the body (12) comprises a mesh having a coating.

8. The endovascular implant of any of claims 2-7, wherein the anchor (14, 16) comprises first and second anchors (14, 16) separated by an inlet to the lumen.

9. The endovascular implant of claim 1, wherein the anchor includes a first anchor (14) extending from a first side of a distal end of the body, and a second anchor (16) extending from a second side of the distal end of the body, the second anchor separated from the first anchor.

## Patentansprüche

1. Endovaskuläres Implantat (10) zum Bilden einer Fistel, umfassend:
einen Körper (12) einschließend eine Wand, die ein Lumen (L) bildet, wobei der Körper ein distales Ende aufweisend eine distale Öffnung und ein proximales Ende aufweisend eine proximale Öffnung einschließt;
einen Anker (14, 16), der mit dem distalen Ende des Körpers angrenzend zur distalen Öffnung gekoppelt ist; und
einen Rückholer (18), der mit dem proximalen Ende des Körpers angrenzend zur proximalen Öffnung gekoppelt ist;
wobei die Wand ein Material aufweisend eine bestimmte Dicke umfasst, und **gekennzeichnet durch** Aufweisen eines Paares miteinander verbundener Haken (18a, 18b), die den Retriever (18) bilden, wobei jeder Haken des Paares von Haken einen ersten longitudinalen Abschnitt aufweist, der sich von der Wand erstreckt und einstückig mit dieser gebildet ist, und einen zweiten Abschnitt, der mit dem ersten longitudinalen Abschnitt verbunden ist, und wobei jeder Haken aus dem Material der Wand aufweisend die Dicke gebildet ist.

2. Endovaskuläres Implantat nach Anspruch 1, wobei der erste longitudinale Abschnitt jedes Hakens teilweise verdreht ist und der zweite Abschnitt radial nach innen vom ersten longitudinalen Abschnitt vorsteht.

3. Endovaskuläres Implantat nach Anspruch 2, wobei das Paar von Haken (18a, 18b) mindestens teilweise durch ein Bindemittel miteinander verbunden ist.

4. Endovaskuläres Implantat nach Anspruch 3, wobei das Bindemittel eine Schweißnaht umfasst.

5. Endovaskuläres Implantat nach Anspruch 2, wobei das Paar miteinander verbundener Haken (18a, 18b) einen radial innersten Abschnitt einschließt, der gegenüber einer Mittelachse des Körpers (12) versetzt ist.

6. Endovaskuläres Implantat nach Anspruch 2, wobei der erste longitudinale Abschnitt jedes Hakens teilweise verdreht ist und der zweite Abschnitt radial nach innen vom ersten longitudinalen Abschnitt vorsteht.

7. Endovaskuläres Implantat nach einem der Ansprüche 2 - 6, wobei der Körper (12) ein Netz umfasst, das eine Beschichtung aufweist.

8. Endovaskuläres Implantat nach einem der Ansprüche 2 - 7, wobei der Anker (14, 16) einen ersten und einen zweiten Anker (14, 16) umfasst, die durch einen Einlass in das Lumen voneinander getrennt sind.

9. Endovaskuläres Implantat nach Anspruch 1, wobei der Anker einen ersten Anker (14), der sich von einer ersten Seite eines distalen Endes des Körpers erstreckt, und einen zweiten Anker (16), der sich von einer zweiten Seite des distalen Endes des Körpers erstreckt, einschließt, wobei der zweite Anker vom ersten Anker getrennt ist.

## Revendications

1. Implant endovasculaire (10) pour former une fistule, comprenant :
un corps (12) incluant une paroi formant une lumière (L), le corps incluant une extrémité distale présentant une ouverture distale et une extrémité proximale présentant une ouverture proximale ;
un ancrage (14, 16) accouplé à l'extrémité distale du corps adjacent à l'ouverture distale ; et
un organe de récupération (18) accouplé à l'extrémité proximale du corps adjacent à l'ouverture proximale ;
dans lequel la paroi comprend un matériau présentant une épaisseur, et **caractérisé en ce qu'**il présente une paire de crochets reliés entre eux (18a, 18b) formant l'organe de récupération (18), chaque crochet de la paire de crochets présentant une première partie longitudinale s'étendant depuis la paroi et formée d'une seule pièce avec celle-ci et une seconde partie reliée à la première partie longitudinale, chaque crochet étant formé du matériau de la paroi présentant l'épaisseur.

2. Implant endovasculaire selon la revendication 1, dans lequel la première partie longitudinale de chaque crochet est partiellement vrillée et la seconde partie s'étend radialement vers l'intérieur depuis la première partie longitudinale.

3. Implant endovasculaire selon la revendication 2, dans lequel la paire de crochets (18a, 18b) sont reliés ensemble au moins partiellement par une liaison.

4. Implant endovasculaire selon la revendication 3, dans lequel la liaison comprend une soudure.

5. Implant endovasculaire selon la revendication 2, dans lequel la paire de crochets reliés entre eux (18a, 18b) incluent une partie radialement la plus interne décalée par rapport à un axe central du corps (12).

6. Implant endovasculaire selon la revendication 2, dans lequel la première partie longitudinale de chaque crochet est partiellement vrillée et la seconde partie s'étend radialement vers l'intérieur depuis la première partie longitudinale.

7. Implant endovasculaire selon l'une quelconque des revendications 2 à 6, dans lequel le corps (12) comprend un maillage présentant un revêtement.

8. Implant endovasculaire selon l'une quelconque des revendications 2 à 7, dans lequel l'ancrage (14, 16) comprend des premier et second ancrages (14, 16) séparés par une entrée vers la lumière.

9. Implant endovasculaire selon la revendication 1, dans lequel l'ancrage inclut un premier ancrage (14) s'étendant depuis un premier côté d'une extrémité distale du corps, et un second ancrage (16) s'étendant depuis un second côté de l'extrémité distale du corps, le second ancrage étant séparé du premier ancrage.
